Europäisches Patentamt

(19) European Patent Office   (11) Publication number:   **0 060 565**

Office européen des brevets   **B1**

(12) **EUROPEAN PATENT SPECIFICATION**

(45) Date of publication of patent specification: **25.06.86**   (51) Int. Cl.⁴: **C 12 N 5/00**

(21) Application number: **82102164.9**

(22) Date of filing: **17.03.82**

(54) **Fully synthetic cell culture medium.**

(30) Priority: **18.03.81 DE 3110559**

(43) Date of publication of application:
**22.09.82 Bulletin 82/38**

(45) Publication of the grant of the patent:
**25.06.86 Bulletin 86/26**

(84) Designated Contracting States:
**AT BE CH DE FR GB IT LI LU NL SE**

(56) References cited:
**US-A-4 205 126**

**HOPPE-SEYLER'S Z. PHYSICL. CHEM., vol. 361, 1980, pages 351-52; J.H. WISSLER et al.: "Leucocyte-derived protein hormones for tissue repair (Lympho-, Mono- and Leucokines): large-scale production, isolation and properties of cell-derived cytotaxins, cytokinesins, cytotoxins and mitogens".**

**CHEMICAL ABSTRACTS, vol. 72, no. 13, 13th March 1970, page 220, no. 65040u, Columbus Ohio (USA); H. OZAWA et al.: "Effect of ubiquinone on respiration of cultured cells (FM3A)".**

(73) Proprietor: **Max-Planck-Gesellschaft zur Förderung der Wissenschaften e.V.
Bunsenstrasse 10
D-3400 Göttingen (DE)**

(72) Inventor: **Wissler, Josef H., Dr.
Liebigstrasse 12
D-6350 Bad Nauheim (DE)**

(74) Representative: **Vossius Vossius Tauchner Heunemann Rauh
Siebertstrasse 4 P.O. Box 86 07 67
D-8000 München 86 (DE)**

Courier Press, Leamington Spa, England.

## Description

For the culture of different cell types, such as bone marrow cells, endothelial cells, and heart muscle cells or leukocytes, various culture media are known. These media normally are aqueous solutions which contain a variety of chemical compounds. Main constituents of these cell culture media are salts, sugars and metabolites, amino acids, nucleotides and nucleosides, vitamins, vitaminoids, coenzymes, steroids and further additives, such as tensides, heavy metal salts and indicator dyes. Special examples of usual culture media are known under the name "HAM", "Medium 199" and "NCTC"; see H. J. Morton, in Vitro 6 (1970), p. 89 to 108.

When culturing cells for more than one hour, as in the case of leukocytes, mostly serum (e.g. fetal calf serum or horse serum) is added to the culture medium. The serum constituents are said to be favourable for the maintenance of cellular functions. However, if the serum-contain culture solution is to be subjected to processes for isolating proteins (mediators) which are formed by culturing cells, the preparation of trace protein products is difficult because of the great number of compounds making up the complex mixture of serum added to the culture. In addition, under such conditions, upon addition of serum to a cell culture medium, it is difficult if not at all impossible to recognize the origin of the mediators: It is then an open question whether or not a specific mediator is of humoral (serum) or cellular (leukocyte) origin and from which species this mediator stems. Thus, the mediator may be derived from the species whose cells have been cultured; or, alternatively, it may be derived from the species from which the added (mostly heterologous) serum stems.

The addition of essential fatty acids (e.g. linoleic, linolenic and arachidonic acids) to usual cell culture media is known from US—A—4,205,126 and from Hoppe-Seyler's Z. Physiol. Chem. Vol. 361 (1980), p. 351. The use of ubiquinone for culturing cells is disclosed in Chem. Abstr. Vol 72, Nr. 13 (1970), p. 220, No. 65040 u.

Besides serum-containing culture media, serum-free, synthetic media are also known; see H. J. Morton, loc. cit; I. Hayashi and G. H. Sato, Nature 259 (1976) p. 132—134; N. N. Iscove and F. Melchers, J. Exp. Med. 147 (1978) p. 923—933.

However, these known media likewise have drawbacks both for the culture of cells and for the preparation of the mediators formed from the culture supernatant. The tensides, heavy metal salts and/or dyes contained therein may damage or irreversibly contaminate the trace mediator proteins.

On the other hand, such known serum-free media are devoid of essential constituents which are necessary for maintaining the structural and functional viability of leukocytes. Therefore, none of the culture media known so far can be suitably used for the culture of leukocytes and the biotechnical preparation of cellular trace components, such as inflammation mediators.

It is therefore a primary object of this invention to provide a new fully synthetic culture medium for the culture of cells, especially of eukaryontic cells, such as leukocytes which provides favourable conditions for the culture of cells and facilitates the isolation from the supernatant culture solution of the proteins secreted by the cells.

The subject matter of the invention is a fully synthetic cell culture medium containing salts, sugars, sugar derivatives, amino acids, amino acid derivatives, nucleosides and nucleoside bases, vitamins, vitaminoids, coenzymes, steroids and other usual additives in aqueous solutions which is characterized in that it additionally contains at least one bio-flavanoid in a concentration of 0.1 to 50 µmol/l and/or vitamin U in a concentration of 0.1 to 20 µmol/l and/or mevalolactone in a concentration of 0.5 to 30 µmol/l.

Preferably the fully synthetic cell culture medium of the invention contains at least two unsaturated fatty acids in a concentration of 0.1 to 20 µmol/l and further also at least one ubiquinone in a concentration of 0.05 to 2.0 µmol/l.

Preferably, the fully synthetic cell culture medium of the invention additionally contains at least one defined protein. In an especially preferred embodiment of the invention, this protein is represented by highly purified, molecularly homogenous serum albumin.

In a further embodiment of the invention, the fully synthetic cell culture medium may contain still further compounds valuable for the culture of eukaryontic cells, such as leukocytes. These compounds belong to the class of polyhydroxy derivatives and sugars, amino acids and peptides, nucleosides and nucleoside bases, anionic compounds and/or vitamins whose use is not usual in known culture media.

Preferably, the cell culture medium of the invention is free of tensides, heavy metal salts and indicator dyes which may be detrimental to cells and may interfere in the isolation of the desired cell products from the supernatant culture solution.

The cell culture medium of the invention is especially useful in the culture of eukaryontic cells, such as bone marrow cells, endothelial cells, heart muscle cells and especially leukocytes, during the culture of which cellular secretory products are produced. The usefulness of the culture medium of the invention stems from its composition of different components which consist of some valuable compounds. They are suitable for preserving the viability of cells and promoting their growth. These types of additional compounds are specified in the following. Their special usefulness in cell culture media has so far not been considered. The cell culture medium of the invention does not damage the cultured cells. On the contrary, it promotes their growth. It makes possible the preparation of cellular secretion products, such as mediator proteins.

2

Apart from cell culture for preparing cellular secretion products, the cell culture medium of the invention is also suitable for cultures which serve for the biological investigation of cells and their functions. Examples of such cultures are the preservation of cell stem lines, investigation of the motility of cells, cell cloning and cell typing.

The cell culture medium is useful for suspension and layer cultures, as well as for cultures in diffusion chambers.

A major advantage of the fully synthetic cell culture medium of the invention consists in the fact that proteins formed by culturing as well as other investigated products can be relatively easily isolated from the supernatant culture solution. This advantage stems from the fact that the culture medium does not contain a complex protein mixture, such as serum, which makes the isolation of cell-secreted products difficult. On the contrary, the culture medium contains only a few defined proteins. A preferred embodiment uses only one single defined protein.

In the following, the components of the inventive culture medium are specified. First, the usual groups of compounds of known media are mentioned.

A first important group of substances in culture media are salts. They include as necessary components, the ions of alkali and alkaline earth metals, such as sodium, potassium magnesium and calcium ions as cations. As anions, chloride, phosphate, sulfate, carbonate and acetate ions are used. The use of salts is necessary in culture media for maintaining cellular functions, e.g. the sodium-potassium membrane transport system.

The second important group of substances in culture media are sugars and sugar derivatives. This group includes as necessary and usual components, for instance glucose, ribose, 2-desoxy-D-ribose and myo-inosite.

Amino acids and their derivatives belong to a third important group of substances in cell culture media. Normally, mixtures of naturally occurring amino acids are used. However, some amino acids, such as L-carnitine and L-ornithine, are not contained in the known culture media. Furthermore, certain peptides are used in culture media, such as L-glutathione.

A further group of substances, which are present in usual culture media, are nucleotides, nucleosides and their derivatives, such as adenine, adenosine, guanine, thymidine, uracil and hypoxanthine.

Finally, usual cell culture media contain different vitamins, vitaminoids and coenzymes, such as ascorbic acid, biotine, pantothenate, ergocalciferol, riboflavine, vitamin A, vitiamin $B_{12}$, pyridoxal and nicotinamide. Furthermore, cholesterol is a usual steroid in culture media.

If necessary, antibiotics are added to known culture media, such as penicillin or streptomycin. Normally, such culture media additionally contain tensides, heavy metal ions and indicator dyes.

The above-mentioned substances are used in a variety of combinations as culture media for the culture of cells. Culture media may be more or less rich in these components. Normally, serum is added to the aqueous solutions of these substances, if a longer cell culture period is necessary. This is especially necessary for culture media poor in components or salt solutions.

The culture medium of the invention is characterized by the following differences compared to known culture media:

1) In addition to the above-mentioned usual mixture of substances, the medium of the invention contains at least one of the following components:

— at least one bioflavanoid,
— vitamin U
— mevalolactone.

Bioflavanoids are derivatives of flavones and known as vitamin P. This group of substances are very valuable constituents of the media for the cells to be cultured. They act on cell membranes in a favourable manner, especially in the case of leukocytes and endothelial cells. Bioflavanoids, such as morine, but especially those containing an L-rutinose or an L-rhamnose residue, such as rutin, hesperdin and quercitrin, are components preferably used in the cell culture medium of the invention. Rutin which is also preferred because of its low price is especially active. The bioflavanoids are present in the cell culture medium of the invention in concentrations of 0.1 to 50 µmol/l, preferably from 1 to 20 µmol/l.

Vitamin U (DL-methionine-methyl sulfonium bromide) is also a very valuable component of the cell culture medium. It promotes the viability of cells. Vitamin U is preferably added in a concentration of 0.1 to 20 µmol/l, preferably from 0.5 to 10 µmol/l.

Mevalolactone (D,L-3,5-dihydroxy-3-methyl-δ-valero-lactone) is an important biochemical compound in the biosynthesis of isoprenoids. Therefore, it was found to be suitable for promoting cell growth. It is added to the medium in a concentration of 0.5 to 30 µmol/l, especially from 1 to 15 µmol/l.

Each of the above-mentioned components which characterize the medium of the invention (bioflavanoid, vitamin U, mevalolactone) provides better conditions for life and growth of the cells, even when only one of these compounds is added. These better conditions for life and growth of the cells in the culture medium are expressed by their viability in the course of the culture. Accordingly, the object of the invention is already achieved if only one of the above-mentioned components is added in addition to the usual constituents of a culture medium. Preferred embodiments of the cell culture medium of the invention contain at least two components freely selected from those mentioned above.

Additionally the culture medium of the invention may contain at least two unsaturated fatty acids and/or at least one ubiquinone.

The unsaturated fatty acids are known as vitamin F. For the cells to be cultured, they are very valuable compounds for the synthesis of complex structures, such as the biologically active prostaglandins, prostacyclins, thromboxans and leukotriens. This especially applies to leukocytes to be cultured. As unsaturated fatty acids, oleic acid, linolic acid and linolenic acid and their derivatives are preferably used.

The cell culture medium of the invention contains at least two of these acids to offer a broad basis of nutrition to the cells. The unsaturated fatty acids in the cell culture medium of the invention are present in concentrations of at least 0.1 µmol/l. Their maximum concentration is 8 times that of the serum albumin concentration in the culture medium. The concentration range of 0.1 to 20 µmol/l of unsaturated fatty acids is preferred.

Ubiquinones are coenzymes, which participate in biological oxidation processes as electron carriers and which play a role in oxidative phosphorylation. This group of substances also represents a very valuable component of the cell culture medium of the invention. Examples of suitable ubiquinones are ubiquinone $Q_{30}$ and ubiquinone $Q_{50}$ (coenzyme $Q_{10}$). The ubiquinones are added to the medium of the invention in a concentration of about 0.01 to 20 µmol/l. The upper concentration of these compounds in the culture medium is limited by the high costs and the limit of their solubility. A concentration range of 0.05 to 2.0 µmol/l is preferred.

Two component combinations of the additives are preferred with one component consisting of at least two unsaturated fatty acids.

Even better culture conditions are provided for the culture by combining several of these special components. Special examples of such favourable combinations of the additional components in the culture medium are:

a) two or three unsaturated fatty acids and one bioflavanoid;
b) the combination according to a)+ubiquinone;
c) the combination according to a)+vitamine U;
d) two or three unsaturated fatty acids, vitamin U and mevalolactone;
e) one bioflavanoid, one ubiquinone and mevalolactone;
f) all possible combinations with four of the components;
g) the combination of all of the 5 components.

2) Being a very complex mixture of substances, serum is said to promote the viability of the cells in the culture. For this reason, it is widely used in usual cell culture media. Although it may have these favourable properties, its use in the medium of the invention is not preferred for the following reasons: If serum is used in the medium for the culture of leukocytes and for the preparation of cellular mediators, humoral mediators are likely to form concomitantly. This would contaminate the investigated cellular products and cause problems in their identification and characterization. It would be highly difficult to determine whether or not a mediator formed and isolated would be of humoral (serum-derived) or cellular origin. In addition, it would be almost impossible to determine the species from which the cellular or humoral mediator stems. The species may be one from which the cells originate or else, it may be the species from which the serum had been taken. Furthermore, it is not preferable to isolate cells from serum and other biological fluids by means of laborious preparation methods, and to contaminate the cells gains with heterologous sera. In addition, if heterologous sera are used in the culture medium, detrimental immune reactions may contribute to loss of cell viability and interfere with secretory reactions.

The addition of serum normally serves to provide cell culture media with proteins and to modify cellular surface properties for optimum chemokinesis of cells. Cells can survive and remain viable in a medium without proteins provided that the period of culture is short. Therefore, in principle, the basic medium of the invention is free of proteins. For this reason it is also suitable for the investigation of chemokinesis and chemotaxis of cells in which the presence of a protein solution may be a disturbing factor. However, in the case of cells which are cultured for longer periods, especially in the case of leukocytes, addition of a protein to the cell culture medium is preferred for several reasons.

Without protein in the cell culture medium, the cells are not motile: They do not show chemikinesis. Furthermore, a protein in culture solutions is necessary as carrier of hydrophobic molecules which, in free form, would be toxic. Thus, in their free form, molecules, such as fatty acids, are soaps. For these reasons, at least one protein is preferably added to the medium. However, to prevent difficulties arising from and becoming apparent by the use of protein mixtures of undefined composition, such as serum, only one defined protein or a mixture of preferably few defined proteins is used. An embodiment in which the cell culture medium of the invention contains only one defined protein is especially preferred.

Special examples of proteins used are serum-albumin, fibrinogen, ovalbumin and similar, defined proteins which are either not capable of forming mediators or contribute only in a specific manner. The preferred protein added to the cell culture medium of the invention is serum albumin. It is not only suitable as carrier of hydrophic substances, but is also biologically active on cell surfaces in a defined manner. For optimum conditions, the protein (serum albumin) is added to the culture solution in a concentration of at least 0.5 µmol/l.

4

0 060 565

Preferably, highly purified, electrophoretically and otherwise molecularly homogenous, human serum-albumin is used. On the one hand human serum albumin is at present the most economic form of highly purified serum-albumin available. On the other hand, for the culture of heterologous cell types, the use of human serum-albumin permits the analysis of its possible influence on and its possible participation in the formation and inactivation of cells and certain cell-secreted mediator substances.

At the preferred low concentration of about 2 to 10 µmol/l serum-albumin confers positive chemokinetic activity to the cells and provides them with some necessary properties which are basic and of paramount importance for cell survival and expression of cellular life functions: The motility, the transport of substances in the membrane and the regulation of cellular adhesion and aggregation tendencies as well as of cellular secretion potencies. Furthermore, this concentration of serum-albumin is high enough and optimally balanced for adsorption of all hydrophobic substances present as basic components or formed and secreted by cells in the medium. Thus, free unsaturated fatty acids, which, as such, are cell-damaging soaps, are adsorbed by the serum-albumin. For this adsorption, one serum-albumin molecule has 8 binding sites for such hydrophobic molecules.

The cell culture medium of the invention may also contain several other proteins as long as these proteins are defined and are either not capable of forming mediators or contribute only in a specific manner.

3) Apart from the above-mentioned characteristics, the medium of the invention can contain further valuable components which are not present in usual media. They include:

— anionic compounds, namely citric acid, succinic acid and/or pyruvic acid. These compounds are valuable nutrients for the viable cells, especially for leukocytes in culture. They are contained in the medium in concentrations from 0.1 to 500, preferably from 0.2 to 100 µmol/l.
— Polyhydrxxy compounds, sugars and sugar derivatives, namely xylitol, D-xylose, D-galactose, glycerin and/or D-glucurono-γ-lactone. These compounds are also valuable for nitrition of cells. They are contained in the medium as single components or as mixture in concentrations from 0.1 to 5, preferably from 0.2 to 2 µmol/l.
— Amino acids and derivatives, namely L-ornithine, sarcosine and taurine. These amino acids and their combinations are not contained in usual cell culture media. But they are very valuable as nutrient components for the culture of leukocytes. The compounds are contained in the medium as single components or as mixture in a concentration of 0.05 to 2, preferably 0.1 to 1 µmol/l.
— L-carnitin and L-carnosin. L-carnitin (3-hydroxy-4-[trimethylammonium-butyric acid-betain) serves as carrier of acyl groups in membranes in the metabolism of fatty acids and has vitamin character. The function of L-carnosin (N-β-alanyl-L-histidine) is so far unknown. However, it is widely distributed in tissues. Both compounds are valuable components for the growth of cells. L-carnitin is added to the medium in in concentrations from 1 to 200, especially from 10 to 100 µmol/l. L-carnosine is added to the medium in concentrations from 0.1 to 20, especially from 1 to 10 µmol/l.
— Nucleosides and nucleoside bases, namely cytidine, guanosine, guanine, 5-methylcytosine and/or uridine. These compounds are also not usual components in normal cell culture media. However, they are necessary especially for cells in the mitotic cycle. These compounds are contained in the medium in concentrations from 0.01 to 1, preferably from 0.02 to 0.5 mmol/l as single components and in admixture.
— Nucleotides are preferably not added to the medium of the invention, even though they are frequently used in normal media. The reason is that nucleotides normally are not transferred to cells. As components of cellular energetics they are synthesized within cells but are not transported as such through membranes. Extracellularly they occur in higher numbers only if cells are damaged.
— Vitamins, namely vitamin $K_1$ (2-methyl-3-phytyl-1,4-naphthoquinone). Vitamin $K_1$ contains the important phytyl residue. Addition of this vitamin results in an especially favourable growth of cells, especially of leukocytes in culture. It is added to the medium of the invention in concentrations of from 0.1 to 2, preferably 0.2 to 1 µmol/l.

4) Preferably, the medium of the invention contains no tensides, no heavy metal salts and no indicator dyes.

Tensides which in normal known culture media presumably serve to dissolve hydrophobic compounds, are not used in the medium of the invention. They may have a detrimental effect on cells and on the structure of cell-secreted proteins, such as mediators. In addition, they are very disturbing factors in the purification behaviour of such proteins.

Heavy metal ions are not added to the cell culture medium of the invention, since they may irreversibly inhibit or denaturate proteins by binding to sulfhydryl groups. At higher concentrations, they are also known to poison cells. Additionally, they may disturb the redox potential of the medium. Heavy metal salts also catalyze the degradation or inactivation of some vitamins, such as vitamin C.

On the other hand, some heavy metal ions are in any case introduced into the medium as contaminants by the other components, in spite of their analytical or pharmaceutical purity degree. Sodium chloride used in its analytical grade for preparation of the medium mainly contributes to the introduction of heavy metal ion contaminants. Usual concentrations of heavy metal contaminants in the medium of the

5

invention are: 0.3 µmol/l iron ions, 0.1 µmol/l lead ions, 0.2 µmol/l copper ions, 0.1 µmol/l cadmium ions, 0.1 µmol/l zinc ions, 0.2 µmol/l aluminum ions, 0.2 µmol/l nickel ions 0.1 µmol/l manganese ions, 0.1 µmol/l cobalt ions, 0.1 µmol/l thallium ions. Other components introduced (arsenate and selenite) are each present at 50 nmol/l as a maximum. In part, such contaminants are also necessary in these low concentrations for cell survival and expression of cellular life functions. Thus, trace amounts of inorganic metal ions are necessary in cellular metabolism and in secretory processes. However, the indicated concentrations in the culture medium, the volume of which is at least about 20 times larger than the wet cell volume are high enough and sufficient for normal promotion of expression of life functions of eukaryons. Thus, the amount of such components introduced as contaminants is large enough for these purposes.

Indicator dyes are disturbing factors, since they may change protein structures by hydrophobic or another type or irreversible binding and suggest false properties of isolated (stained) biologically active trace proteins.

The ratios of the components of the cell culture medium of the invention are balanced so that their concentrations in aqueous solutions largely correspond to the natural concentration ratios of the same components in natural blood plasma or serum; see Ciba Geigy AG (eds.) 1969 (in Documenta Geigy, Wissenschaftliche Tabellen, 7th edition, Geigy SA Basle). The principle of balancing the components according to their plasma concentrations is departed from in the preparation of the medium only for economic reasons. Thus, the expensive serum albumin is preferably added in a low but high enough concentration for not impairing the chemokinesis of cells. For the culture of the cells, further additional components can be added to the medium of the invention, if a certain purpose requires such additional components. Thus, for the stimulation of physiological functions of cells, hormones, such as insulin, glucagon, chemorecruitins, etc., and mediators, such as chemokinesins, chemotaxins or chemotropins, and mitogens, also in the form of polyvalent lectin mitogens can be added.

Furthermore, apart from the usual phosphate and carbonate buffer systems in the medium, other buffer systems can be used in addition to or instead of the mentioned inorganic buffers. Thus, for example, organic buffer systems such as HEPES (N-2-hydroxy-ethyl)-piperazine-N'-2-ethane sulfonic acid) can be used in a usual concentration of 0.01 mol/l.

In the following Table I, the components of a usual cell culture medium are summarized. With respect to its components, the medium summarized in Table I largely corresponds to the known medium 199, except that tensides, heavy metal salts, indicator dyes and nucleotides are omitted. These latter components are preferably omitted from the medium of the invention.

Furthermore, however, the concentrations of the components of the basic mixture according to Table I do not correspond to those of the medium 199. But they are given in Table I according to the purpose of the medium of the invention, i.e. they are balanced to the corresponding natural concentration of the components of the plasma.

The medium composition according to Table I represents a basic mixture also for the media of the invention specified in the following examples. This means that in the examples—unless otherwise stated—only the additional components are given in the corresponding tables.

For the production of the medium, water of ASTM-1 quality is exclusively used; see ASTM D-1193-70 Standard Specification for Reagent Water 1970; Annual Book of ASTM-Standards, Easton Maryland, ASTM 1970. In addition, this high quality water is freed from possible endotoxin contaminants by ultrafiltration at tenside-free membranes with an exclusion limit of 10,000 daltons. The finished medium is sterilized by filtration at tenside-free membranes with a pore size ≤0.2 µm.

TABLE I

| No. | Component | mol/l | No. | Component | mol/l |
|---|---|---|---|---|---|
| 1 | KCl | 5,0m | 10 | myo-Inositol | 0,5m |
| 2 | KH$_2$PO$_4$ | 0,2m | 11 | Na-acetate | 0,2m |
| 3 | NaCl | 120,0m | 12 | D-Ribose | 20,0µ |
| 4 | Na$_2$HPO$_4$ | 0,8m | 13 | CaCl$_2$ | 2,0m |
| 5 | Na$_2$SO$_4$ | 0,2m | 14 | MgCl$_2$ | 1,0m |
| 6 | L-Ascorbic acid | 0,2m | 15 | NaHCO$_3$ | 10,0 |
| 7 | Choline chloride | 50,0µ | 16 | Penicillin | 1,0µ |
| 8 | 2-Desoxy-D-ribose | 5,0µ | 17 | Streptomycin | 1,0µ |
| 9 | D-Glucose | 5,0m | 18 | L-Glutamine | 1,0m |

6

# 0 060 565

TABLE I (contd.)

| No. | Component | mol/l | No. | Component | mol/l |
|---|---|---|---|---|---|
| 19 | L-Alanine | 0,2m | 43 | Guanine | 5,0μ |
| 20 | L-Asparagine | 0,1m | 44 | Hypoxanthine | 5,0μ |
| 21 | L-Aspartic acid | 0,1m | 45 | Thymidine | 20,0μ |
| 22 | L-Glutamic acid | 0,1m | 46 | Thymine | 5,0μ |
| 23 | Glycine | 0,2m | 47 | Uracil | 5,0μ |
| 24 | L-Proline | 0,1m | 48 | Xanthine | 5,0μ |
| 25 | L-Serine | 0,1m | 49 | D-Biotin | 1,0μ |
| 26 | L-Arginine | 0,1m | 50 | D-Ca-pantothenate | 5,0μ |
| 27 | 4-aminobenzoic acid | 2,0μ | 51 | Ergocalciferol | 0,5μ |
| 28 | L-Cysteine | 0,2m | 52 | Folic acid | 5,0μ |
| 29 | L-Histidine | 0,1m | 53 | D,L-α-lipoic acid | 2,0μ |
| 30 | L-Hydroxyproline | 10,0μ | 54 | Menadion | 0,2μ |
| 31 | L-Isoleucine | 0,2m | 55 | Nicotinic acid amide | 20,0μ |
| 32 | L-Leucine | 0,2m | 56 | Pyridoxal-HCl | 5,0μ |
| 33 | L-Lysin-HCl | 0,2m | 57 | Pyridoxine-HCl | 2,0μ |
| 34 | L-Methionine | 0,1m | 58 | Riboflavin | 1,0μ |
| 35 | L-Phenylalanine | 0,1m | 59 | Thiamine-HCl | 5,0μ |
| 36 | L-Threonine | 0,2m | 60 | D,L-α-tocopheryl acetate | 1,0μ |
| 37 | L-Tryptophane | 50,0μ | 61 | Vitamin-A-acetate | 1,0μ |
| 38 | L-Tyrosine | 0,1m | 62 | Vitamin $B_{12}$ | 0,5μ |
| 39 | L-Valine | 0,2m | 63 | Cholesterol | 1,0μ |
| 40 | Glutathion reduced | 3,0μ | 64 | Ethanol | 1,0m |
| 41 | Adenine | 50,0μ | 65 | pH 7,10 | — |
| 42 | Adenosine | 50,0μ | | | |

Example 1

A cell culture medium is prepared which in addition to the components of Table I contains the following constituents:

7

## 0 060 565

TABLE II

| Component | Amount, µmol/l |
|---|---|
| Linoleic acid | 1,0 |
| Linolenic acid | 5,0 |
| Oleic acid | 5,0 |
| Rutin | 5,0 |
| Vitamin U | 1,0 |
| D-Galactose | 500,0 |
| Succinic acid | 100,0 |
| D-Xylose | 20,0 |
| L-Ornithine | 50,0 |
| Sarcosine | 50,0 |
| Guanosine | 20,0 |
| D,L-Carnitine | 50,0 |

In the medium of Table II which contains these components in addition to the basic mixture of Table I, $10^6$ lymphocytes per ml from dog spleen were cultured. The culture is performed under usual sterile conditions (37°C, 3% $CO_2$) as a layer culture. For stimulation of mitosis in the cells, 50 nmol/l lectin from Canavalia ensiformis (Concanavalin A, CON) are added. After 48 hours the cells are counted and subjected to chromosome analysis. The number of viable cell is higher than 90%. The main portion of the cells has undergone mitosis.

Example 2

A cell culture medium is prepared which in addition to the basic mixture of components of Table I contains the following constituents given in Table III.

TABLE III

| Component | Amount, µmol/l |
|---|---|
| Linoleic acid | 1,5 |
| Linolenic acid | 4,0 |
| Oleic acid | 5,0 |
| Coenzyme $Q_{10}$ | 0,1 |
| Mevalolactone | 5,0 |
| Glycerol | 50,0 |
| Xylitol | 10,0 |
| 5-Methylcytosine | 5,0 |
| Carnosine | 5,0 |

The culture of lymphocytes is performed according to example 1 and under the same conditions, in the basic mixture medium with the additional components of Table III. Corresponding results as to the viability of cells and mitosis are obtained.

8

Example 3

A cell culture medium is prepared which in addition to the basic mixture of components of Table I contains the following constituents given in Table IV.

TABLE IV

| Component | Amount, µmol/l |
|---|---|
| Mevalolactone | 5,0 |
| D-Glucurono-γ-lactone | 100,0 |
| Taurine | 0,1 |
| D,L-Carnitine | 50,0 |
| Vitamin $K_1$ | 0,2 |
| Human serum albumin | 30,0 |

A fibroblast monolayer culture (mouse L NCTC Clone 929) is seeded into the medium. The conditions correspond to those given in example 1. After termination of the culture, the cells show a viability of >92%.

Example 4

Example 3 is performed with the modification that instead of mevalolactone the same concentration of morine is added. The viability of cells after culture is >90%.

Example 5

Example 3 is repeated with the modification that instead of mevalolactone, vitamin U is added in a concentration of 2 µmol/l. The viability of the cells after culture is >90%.

Example 6

Example 3 is repeated with the modification that in addition linolic acid, linolenic acid, quercitrine and coenzyme $Q_{10}$ are added in concentrations of 2.0, 5.0, 3.0 and 0.2 µmol/l, respectively. The viability of the cells after culture is >95%.

Example 7

For the biotechnical and analytical production and purification of cellular, leukocyte-derived mediators, leukocytes are cultured in a preferred embodiment of the cell culture medium of the invention which is especially rich in components. The composition of the medium according to this preferred embodiment is given in Table V. The medium contains 91 components which are listed according to their solubility characteristics, in order to exemplify a possible mode of preparation of the preferred medium and its stock solutions. In addition, this Table V also exemplifies the possible addition, for special purposes, of further components listed as components 92—94 to the medium.

Thus, for the special purpose of suppressing possible microbial growth during culture, components 92 and 93 (antibiotics in a preferred concentration range of >1 µmol/l) are added. In addition, for the special purpose of stimulating cells mitogenically, component 94 is added, e.g. in the form of a polyvalent lectin mitogen from Canavalia ensiformis (Concanavalin A, CON).

9

TABLE V

| No. | Component | mol/l | No. | Component | mol/l |
|---|---|---|---|---|---|
| 1 | Disodium hydrogen-phosphate | 0.8m | 30 | D-Biotine (Vitamin H) | 1.0μ |
| 2 | Potassium dihydrogen-phosphate | 0.2m | 31 | Cytidine | 50.0μ |
| | | | 32 | L-Glutamic acid | 0.1m |
| 3 | Potassium chloride | 5.0m | 33 | L-Isoleucine | 0.2m |
| 4 | Sodium chloride | 120.0m | 34 | 5-Methylcytosine | 5.0μ |
| 5 | Sodium sulfate | 0.2m | 35 | L-Phenylalanine | 0.1m |
| 6 | D-Glucose | 5.0m | 36 | Riboflavine (B2) | 1.0μ |
| 7 | L-Ascorbic acid (C) | 0.2m | 37 | Thymine (5-methyl-uracil) | 5.0μ |
| 8 | Choline chloride | 50.0μ | 38 | L-Tryptophane | 50.0μ |
| 9 | 2-Deoxy-D-ribose | 5.0μ | 39 | L-Tyrosine | 0.1m |
| 10 | D-Galactose | 0.5m | 40 | Uracil | 5.0μ |
| 11 | D-Glucurono-γ-lactone | 0.1m | 41 | Uridine | 20.0μ |
| 12 | Glycerol | 50.0μ | 42 | L-Leucine | 0.2m |
| 13 | Myo-inositol | 0.5m | 43 | L-Valine | 0.2m |
| 14 | Sodium acetate | 0.2m | 44 | Thymidine | 20.0μ |
| 15 | Sodium citrate | 50.0μ | 45 | Water | 55.4 |
| 16 | Sodium pyruvate | 0.1m | 46 | Hydrogen ions (pH 7.1) | 79.4n |
| 17 | D-Ribose | 20.0μ | 47 | Oxygen (air satura-tion) | 0.2m |
| 18 | Succinic acid | 0.1m | | | |
| 19 | Xylitol | 10.0μ | 48 | L-Alanine | 0.2m |
| 20 | D-Xylose | 20.0μ | 49 | L-Arginine | 0.1m |
| 21 | Calcium chloride | 2.0m | 50 | D,L-Carnithine chloride (BT) | 50.0μ |
| 22 | Magnesium chloride | 1.0m | 51 | L-Carnosine | 5.0μ |
| 23 | Sodium hydrogen-carbonate | 10.0m | 52 | L-Cysteine | 0.2m |
| 24 | Serum albumin (human) | 7.7μ | 53 | L-Blutathione reduced | 3.0μ |
| 25 | L-Asparagine | 0.1m | 54 | Glycine | 0.2m |
| 26 | L-Glutamine | 1.0m | 55 | L-Histidine | 0.1m |
| 27 | Adenosine | 50.0μ | 56 | L-Hydroxyproline | 10.0μ |
| 28 | 4-Aminobenzoic acid | 2.0μ | 57 | L-Lysine-HCl | 0.2m |
| 29 | L-Aspartic acid | 0.1m | 58 | L-Methionine | 0.1m |

TABLE V (contd.)

| No. | Component | mol/l | No. | Component | mol/l |
|---|---|---|---|---|---|
| 59 | D,L-Mevalolactone | 5.0μ | 78 | Rutin (Vitamin P) | 5.0μ |
| 60 | Nicotinic acid amide | 20.0μ | 79 | Xanthine | 5.0μ |
| 61 | L-Ornithine-HCl | 50.0μ | 80 | Ethanol (60 μl/l) | 1.0m |
| 62 | D-Ca-pantothenate (B5) | 5.0μ | 81 | Cholesterol | 1.0μ |
| 63 | L-Proline | 0.1m | 82 | Ergocalciferol (D2) | 0.5μ |
| 64 | Pyridoxal-HCl | 5.0μ | 83 | D,L-α-lipoic acid | 2.0μ |
| 65 | Pyridoxine-HCl (B6) | 2.0μ | 84 | Menadione (K3) | 0.2μ |
| 66 | Sarcosine | 50.0μ | 85 | D,L-α-tocopherol acetate (E) | 1.0μ |
| 67 | L-Serine | 0.1m | | | |
| 68 | Taurine | 0.1m | 86 | Coenzyme Q10 ubi-quinone 50 | 0.1μ |
| 69 | Thiamine-HCl (B1) | 5.0μ | 87 | 3-Phytylmenadione (K1) | 0.2μ |
| 70 | L-Threonine | 0.2m | 88 | Retinol acetate (A) | 1.0μ |
| 71 | Vitamin B12 | 0.5μ | 89 | Linolenic acid (F) | 5.0μ |
| 72 | Vitamin U | 1.0μ | 90 | Linoleic acid (F) | 1.0μ |
| 73 | Adenine | 50.0μ | 91 | Oleic acid | 5.0μ |
| 74 | Folic acid (Bc) | 5.0μ | 92 | Penicillin G | 80.0μ |
| 75 | Guanine | 5.0μ | 93 | Streptomycin | 80.0μ |
| 76 | Guanosine | 20.0μ | 94 | Activator(s) (CON A) | 50.0n |
| 77 | Hypoxanthine | 5.0μ | | | |

The groups of compounds in Table V indicate the compositions of the stock solutions (20—20,000 fold concentrations): components Nos. 1—26, 45—72 and 92—94 are soluble in water at 37°C; components Nos. 27—44 are soluble in water at 90°C; components Nos. 73—79 are soluble in 0.01 mol/l NaOH, pH 12.0 at 37°C; components Nos. 80—91 are soluble in warm ethanol.

During culture, the glucose level as well as the pH value and the oxygen concentration may be measured and kept constant. Thus, the constant glucose level remains within the constant physiological plasma concentration range of glucose. Therefore, a hormone regulation of glucose levels is not necessary.

The medium has a balanced sulfhydryl-group preserving redox potential which also maintains sensitive disulfide bridges in proteins and cell surfaces.

The preparation and culture of leukocytes occurs under sterile conditions. The culture is preferably performed for 40 hours to obtain a satisfactory mediator production. The temperature of the culture is 37°C, the cell concentration is about $10^6$ to $5 \times 10^7$ cell/ml and the $CO_2$ partial pressure in the culture surrounding atmosphere is 1.5%. After terminating the culture, the leukocytes are centrifuged off from the supernatant culture solution. Their viability is larger than 98%. The cells can be again subcultured, cryo-preserved or used for another (biochemical) purpose.

Example 8

In the cell culture medium of example 7 (Table V) with otherwise same conditions, endothelial cells of porcine aorta are cultured as monolayer and second passage subculture. The addition of leukocyte-derived angiotropins induces numerous mitoses in the cells.

# 0 060 565

**Claims for the Contracting States: BE, CH, DE, FR, GB, IT, LI, LU, NL, SE**

1. A fully synthetic cell culture medium containing salts, sugars, sugar derivatives, amino acids, amino acid derivatives, nucleosides and nucleoside bases, vitamins, vitaminoids, coenzymes, steroids and other usual additives in aqueous solution, characterized in that it additionally contains at least one bioflavanoid in a concentration of 0.1 to 50 $\mu$mol/l and/or vitamin is in a concentration of 0.1 to 20 $\mu$mol/l and/or mevalolactone in a concentration of 0.5 to 30 $\mu$mol/l.

2. The medium according to claim 1, characterized in that it further contains at least two unsaturated fatty acids in a concentration of 0.1 to 20 $\mu$mol/l.

3. The medium according to claims 1 or 2, characterized in that it further contains at least one ubiquinone in a concentration of 0.05 to 2.0 $\mu$mol/l.

4. The medium according to claims 1 to 3, characterized in that it further contains at least one defined protein.

5. The medium according to claim 4, characterized in that it contains highly purified, molecularly homogeneous serum albumin.

6. The medium according to claims 1 and 5, characterized in that it additionally contains at least one of the substances: xylitol, D-xylose, D-galactose, glycerol and glucurono-$\gamma$-lactone and/or

at least one of the substances: L-ornithine, sarcosine and taurine, and/or

at least one of the substances: D,L-carnitine and L-carnosine, and/or

at least one of the substances: cytidine, guanosine, 5-methylcytosine and uridine, and/or

at least one of the substances: citric acid, succinic acid and pyruvic acid, and/or

vitamin $K_1$.

7. The medium according to claims 1 to 6, characterized in that it contains the constituents in a quantitative ratio adjusted to the composition of natural blood plasma.

8. The medium according to claims 1 to 7, characterized in that it is free of tensides, heavy metal salts and dyes.

9. The use of the cell culture medium according to claims 1 to 8 for the culture of eukaryontic cells, preferably leukocytes from mammals.

**Claims for the Contracting State: AT**

1. A process for preparing a fully synthetic cell culture medium containing salts, sugars, sugar derivatives, amino acids, amino acid derivatives, nucleosides and nucleoside bases, vitamins, vitaminoids, coenzymes, steroids and other usual additives in aqueous solution characterized in that at least one bioflavanoid in a concentration of 0.1 to 50 $\mu$mol/l and/or vitamin U is in a concentration of 0.1 to 20 $\mu$mol/l and/or mevalolactone in a concentration of 0.5 to 30 $\mu$mol/l is added to said cell culture medium.

2. The process according to claim 1, characterized in that further at least two unsaturated fatty acids are added in a concentration of 0.1 to 20 $\mu$mol/l.

3. The process according to claims 1 or 2, characterized in that further at least one ubiquinone is added in a concentration of 0.05 to 2.0 $\mu$mol/l.

4. The process according to claims 1 to 3, characterized in that further at least one defined protein is added.

5. The process according to claim 4, characterized in that highly purified, molecularly homogenous serum albumin is added.

6. The process according to claims 1 and 5, characterized in that additionally at least one of the substances: xylitol, D-xylose, D-galactose, glycerol and glucurono-$\gamma$-lactone and/or

at least one of the substances: L-ornithine, sarcosine and taurine, and/or

at least one of the substances: D,L-carnitine and L-carnosine, and/or

at least one of the substances: cytidine, guanosine, 5-methylcytosine and uridine, and/or

at least one of the substances: citric acid, succinic acid and pyruvic acid, and/or

vitamin $K_1$ is added.

7. A fully synthetic cell culture medium containing salts, sugars, sugar derivatives, amino acids, amino acid derivatives, nucleosides and nucleoside bases, vitamins, vitaminoids, coenzymes, steroids and other usual additives in aqueous solution, characterized in that it additionally contains at least one bioflavanoid in a concentration of 0.1 to 50 $\mu$mol/l and/or vitamin U in a concentration of 0.1 to 20 $\mu$mol/l and/or mevalolactone in a concentration of 0.5 to 30 $\mu$mol/l.

8. The medium according to claim 7, characterized in that it further contains at least two unsaturated fatty acids in a concentration of 0.1 to 20 $\mu$mol/l.

9. The medium according to claims 7 or 8 characterized in that it further contains at least one ubiquinone in a concentration of 0.05 to 2.0 $\mu$mol/l.

10. The medium according to claims 7 to 9, characterized in that it further contains at least one defined protein.

11. The medium according to claim 10, characterized in that it contains highly purified, molecularly homogenous serum albumin.

12

12. The medium according to claims 7 and 11, characterized in that it additionally contains at least one of the substances: xylitol, D-xylose, D-galactose, glycerol and glucurono-γ-lactone and/or
at least one of the substances: L-ornithine, sarcosine and taurine, and/or
at least one of the substances: D,L-carnitine and L-carnosine, and/or
at least one of the substances: cytidine, guanosine, 5-methylcytosine and uridine, and/or
at least one of the substances: citric acid, succinic acid and pyruvic acid, and/or
vitamin $K_1$.

13. The medium according to claims 7 to 12, characterized in that it contains the constituents in a quantitative ratio adjusted to the composition of natural blood plasma.

14. The medium according to claims 7 to 13, characterized in that it is free of tensides, heavy metal salts and dyes.

15. The use of the cell culture medium according to claims 7 to 14 for the culture of eukaryontic cells, preferably leukocytes from mammals.

**Patentansprüche für die Vertragsstaaten: BE, CH, DE, FR, GB, IT, LI, LU, NL, SE**

1. Vollsynthetisches Zellkulturmedium, enthaltend Salze, Zucker, Zuckerderivate, Aminosäuren, Aminosäurederivate, Nucleoside und Nucleosidbasen, Vitamine, Vitaminoide, Coenzyme, Steroide und andere übliche Zusätze in wäßriger Lösung, dadurch gekennzeichnet, daß es zusätzlich mindestens ein Bioflavanoid in einer Konzentration von 0,1 bis 50 µMol/Liter und/oder Vitamin U in einer Konzentration von 0,1 bis 20 µMol/Liter und/oder Mevalolacton in einer Konzentration von 0,5 bis 30 µMol/Liter enthält.

2. Medium nach Anspruch 1, dadurch gekennzeichnet, daß es zusätzlich mindestens zwei ungesättigte Fettsäuren in einer Konzentration von 0,1 bis 20 µMol/Liter enthält.

3. Medium nach den Ansprüchen 1 oder 2, dadurch gekennzeichnet, daß es zusätzlich mindestens ein Ubichinon in einer Konzentration von 0,05 bis 2,0 µMol/Liter enthält.

4. Medium nach den Ansprüchen 1 bis 3, dadurch gekennzeichnet, daß es zusätzlich mindestens ein definiertes Protein enthält.

5. Medium nach Anspruch 4, dadurch gekennzeichnet, daß es hochreines, molekular homogenes Serum-Albumin enthält.

6. Medium nach den Ansprüchen 1 und 5, dadurch gekennzeichnet, daß es zusätzlich mindestens einen der Stoffe:
Xylit, D-Xylose, D-Galactose, Glycerin und Glukurono-γ-Lacton und/oder
mindestens einen der Stoffe: L-Ornithin, Sarcosin und Taurin und/oder
mindestens einen der Stoffe: D,L-Carnitin und L-Carnosin, und/oder
mindestens einen der Stoffe: Cytidin, Guanosin, 5-Methylcytosin und Uridin, und/oder
mindestens einen der Stoffe: Citronensäure, Bernsteinsäure und Pyruvinsäure, und/oder
Vitamin $K_1$ enthält.

7. Medium nach den Ansprüchen 1 bis 6, dadurch gekennzeichnet, daß es die Bestandteile in einem auf die Zusammensetzung von natürlichem Blutplasma abgestimmten quantitativen Verhältnis enthält.

8. Medium nach den Ansprüchen 1 bis 7, dadurch gekennzeichnet, daß es frei von Tensiden, Schwermetallsalzen und Farbstoffen ist.

9. Verwendung des Zellkulturmediums nach den Ansprüchen 1 bis 8 zur Kultivierung von eukaryontischen Zellen, vorzugsweise Leukozyten von Säugern.

**Patentansprüche für den Vertragsstaat: AT**

1. Verfahren zur Herstellung eines Salze, Zucker, Zuckerderivate, Aminosäuren, Aminosäurederivate, Nucleoside und Nucleosidbasen, Vitamine, Vitaminoide, Coenzyme, Steroide und andere übliche Zusätze in wäßriger Lösung enthaltenden vollsynthetischen Zellkulturmediums, dadurch gekennzeichnet, daß man dem genannten Zellkulturmedium mindestens ein Bioflavanoid in einer Konzentration von 0,1 bis 50 µMol/Liter und/oder Vitamin U in einer Konzentration von 0,1 bis 20 µMol/Liter und/oder Mevalolacton in einer Konzentration von 0,5 bis 30 µMol/Liter zusetzt.

2. Verfahren nach Anspruch 1, dadurch gekennzeichnet, daß man zusätzlich mindestens zwei ungesättigte Fettsäuren in einer Konzentration von 0,1 bis 20 µMol/Liter zusetzt.

3. Verfahren nach den Ansprüchen 1 oder 2, dadurch gekennzeichnet, daß man zusätzlich mindestens ein Ubichinon in einer Konzentration von 0,05 bis 2,0 µMol/Liter zusetzt.

4. Verfahren nach den Ansprüchen 1 bis 3, dadurch gekennzeichnet, daß man zusätzlich mindestens ein definiertes Protein zusetzt.

5. Verfahren nach Anspruch 4, dadurch gekennzeichnet, daß man hochreines, molekular homogenes Serum-Albumin zusetzt.

6. Medium nach den Ansprüchen 1 und 5, dadurch gekennzeichnet, daß man zusätzlich mindestens einen der Stoffe: Xylit, D-Xylose, D-Galactose, Glycerin und Glukurono-γ-Lacton und/oder
mindestens einen der Stoffe: L-Ornithin, Sarcosin und Taurin und/oder
mindestens einen der Stoffe: D,L-Carnitin und L-Carnosin, und/oder
mindestens einen der Stoffe: Cytidin, Guanosin, 5-Methylcytosin und Uridin, und/oder

**0 060 565**

mindestens einen der Stoffe: Citronensäure, Bernsteinsäure und Pyruvinsäure, und/oder Vitamin $K_1$ zusetzt.

7. Vollsynthetisches Zellkulturmedium, enthaltend Salze, Zucker, Zukkerderivate, Aminosäuren, Aminosäurederivate, Nucleoside und Nucleosidbasen, Vitamine, Vitaminoide, Coenzyme, Steroide und andere übliche Zusätze in wäßriger Lösung, dadurch gekennzeichnet, daß es zusätzlich mindestens ein Bioflavanoid in einer Konzentration von 0,1 bis 50 µMol/Liter und/oder Vitamin U in einer Konzentration von 0,1 bis 20 µMol/Liter und/oder Mevalolacton in einer Konzentration von 0,5 bis 30 µMol/Liter enthält.

8. Medium nach Anspruch 7, dadurch gekennzeichnet, daß es zusätzlich mindestens zwei ungesättigte Fettsäuren in einer Konzentration von 0,1 bis 20 µMol/Liter enthält.

9. Medium nach den Ansprüchen 7 oder 8, dadurch gekennzeichnet, daß es zusätzlich mindestens ein Ubichinon in einer Konzentration von 0,05 bis 2,0 µMol/Liter enthält.

10. Medium nach den Ansprüchen 7 bis 9, dadurch gekennzeichnet, daß es zusätzlich mindestens ein definiertes Protein enthält.

11. Medium nach Anspruch 10, dadurch gekennzeichnet, daß es hochreines, molekular homogenes Serum-Albumin enthält.

12. Medium nach den Ansprüchen 7 und 11, dadurch gekennzeichnet, daß es zusätzlich mindestens einen der Stoffe: Xylit, D-Xylose, D-Galactose, Glycerin und Glukurono-γ-Lacton und/oder

mindestens einen der Stoffe: L-Ornithin, Sarcosin und Taurin, und/oder

mindestens einen der Stoffe: D,L-Carnitin und L-Carnosin, und/oder

mindestens einen der Stoffe: Cytidin, Guanosin, 5-Methylcytosin und Uridin, und/oder

mindestens einen der Stoffe: Citronensäure, Bernsteinsäure und Pyruvinsäure, und/oder

Vitamin $K_1$ enthält.

13. Medium nach den Ansprüchen 7 bis 12, dadurch gekennzeichnet, daß es die Bestandteile in einem auf die Zusammensetzung von natürlichem Blutplasma abgestimmten quantitativen Verhältnis enthält.

14. Medium nach den Ansprüchen 7 bis 13, dadurch gekennzeichnet, daß es frei von Tensiden, Schwermetallsalzen und Farbstoffen ist.

15. Verwendung des Zellkulturmediums nach den Ansprüchen 7 bis 14 zur Kultivierung von eurkaryontischen Zellen, vorzugsweise Leukozyten von Säugern.

**Revendications pour les Etats Contractants: BE, CH, DE, FR, GB, IT, LI, LU, NL, SE**

1. Milieu de culture cellulaire totalement synthétique contenant des sels, des sucres, des dérivés de sucres, des amino-acides, des dérivés d'amino-acides, des nucléosides et des bases nucléosidiques, des vitamines, des vitaminoïdes, des coenzymes, des stéroïdes et d'autres additifs usuels en solution aqueuse, caractérisé en ce qu'il contient de plus au moins un bioflavanoïde en une concentration de 0,1 à 50 µmoles/litre et/ou de la vitamine U en une concentration de 0,1 à 20 µmoles/litre et/ou de la mévalolactone en une concentration de 0,5 à 30 µmoles/litre.

2. Milieu suivant la revendication 1, caractérisé en ce qu'il contient en outre au moins deux acides gras insaturés en une concentration de 0,1 à 20 µmoles/litre.

3. Milieu suivant l'une ou l'autre des revendications 1 et 2, caractérisé en ce qu'il contient en outre au moins une ubiquinone en une concentration de 0,05 à 2,0 µmoles/litre.

4. Milieu suivant l'une quelconque des revendications 1 à 3, caractérisé en ce qu'il contient en outre au moins une protéine définie.

5. Milieu suivant la revendication 4, caractérisé en ce qu'il contient de l'albumine de sérum moléculairement homogène, hautement purifiée.

6. Milieu suivant l'une quelconque des revendications 1 et 5, caractérisé en ce qu'il contient de plus au moins une des substances: xylitol, D-xylose, D-galactose, glycérol et glycurono-γ-lactone et/ou

au moins une des substances: L-ornithine, sarcosine et taurine, et/ou

au moins une des substances: D,L-carnitine et L-carnosine, et/ou

au moins une des substances: cytidine, guanosine, 5-méthylcytosine et uridine, et/ou

au moins une des substances: acide citrique, acide succinique et acide pyruvique, et/ou

de la vitamine $K_1$.

7. Milieu suivant l'une quelconque des revendications 1 à 6, caractérisé en ce qu'il contient les constituants en un rapport quantitatif ajusté à la composition de plasma sanguin naturel.

8. Milieu suivant l'une quelconque des revendications 1 à 7, caractérisé en ce qu'il est exempt d'agents tensio-actifs, de sels de métaux lourds et de colorants.

9. Utilisation du milieu de culture cellulaire suivant l'une quelconque des revendications 1 à 8, pour la culture de cellules eucaryotiques, de préférence de leucocytes de mammifères.

**Revendications pour l'Etat Contractant: AT**

1. Procédé de préparation d'un milieu de culture cellulaire totalement synthétique contenant des sels, des sucres, des dérivés de sucres, des amino-acides, des dérivés d'amino-acides, des nucléosides et des bases nucléosidiques, des vitamines, des vitaminoïdes, des coenzymes, des stéroïdes, et d'autres additifs usuels en solution aqueuse, caractérisé en ce que l'on ajoute au moins un bioflavanoïde et une

14

concentration de 0,1 à 50 μmoles/litre et/ou de la vitamine U en une concentration de 0,1 à 20 μmoles/litre et/ou de la mévalolactone en une concentration de 0,5 à 30 μmoles/litre à ce milieu de culture cellulaire.

2. Procédé suivant la revendication 1, caractérisé en ce que l'on ajoute en outre au moins deux acides gras insaturés en une concentration de 0,1 à 20 μmoles/litre.

3. Procédé suivant l'une ou l'autre des revendications 1 et 2, caractérisé en ce que l'on ajoute en outre au moins une ubiquinone en une concentration de 0,05 à 2,0 μmoles/litre.

4. Procédé suivant l'une quelconque des revendications 1 à 3, caractérisé en ce que l'on ajoute en outre au moins une protéine définie.

5. Procédé suivant la revendication 4, caractérisé en ce que l'on ajoute de l'albumine de sérum moléculairement homogène, hautement purifiée.

6. Procédé suivant l'une quelconque des revendications 1 et 5, caractérisé en ce que l'on ajoute en plus au moins une des substances: xylitol, D-xylose, D-galactose, glycérol et glycurono-γ-lactone et/ou
au moins une des substances: L-ornithine, sarcosine et taurine, et/ou
au moins une des substances: D,L-carnitine et L-carnosine, et/ou
au moins une des substances: cytidine, guanosine, 5-méthylcytosine et uridine, et/ou
au moins une des substances: acide citrique, acide succinique et acide pyruvique, et/ou
de la vitamine K$_1$.

7. Milieu de culture cellulaire totalement synthétique contenant des sels, des sucres, des dérivés de sucres, des amino-acides, des dérivés d'amino-acides, des nucléosides et des bases nucléosidiques, des vitamines, des vitaminoïdes, des coenzymes, des stéroïdes et d'autres additifs usuels en solution aqueuse, caractérisé en ce qu'il contient de plus au moins un bioflavanoïde en une concentration de 0,1 à 50 μmoles/litre et/ou de la vitamine U en une concentration de 0,1 à 20 μmoleu/litre et/ou de la mévalolactone en une concentration de 0,5 à 30 μmoles/litre.

8. Milieu suivant la revendication 7, caractérisé en ce qu'il contient en outre au moins deux acides gras insaturés en une concentration de 0,1 à 20 μmoles/litre.

9. Milieu suivant l'une ou l'autre des revendications 7 et 8, caractérisé en ce qu'il contient en outre au moins une ubiquinone en une concentration de 0,05 à 2,0 μmoles/litre.

10. Milieu suivant l'une quelconque des revendications 7 à 9, caractérisé en ce qu'il contient en outre au moins une protéine définie.

11. Milieu suivant la revendication 10, caractérisé en ce qu'il contient de l'albumine de sérum moléculairement homogène, hautement purifiée.

12. Milieu suivant l'une quelconque des revendications 7 et 11, caractérisé en ce qu'il contient de plus au moins une des substances: xylitol, D-xylose, D-galactose, glycérol et glycurono-γ-lactone et/ou
au moins une des substances: L-ornithine, sarcosine et taurine, et/ou
au moins une des substances: D,L-carnitine et L-carnosine, et/ou
au moins une des substances: cytidine, guanosine, 5-méthylcytosine et uridine, et/ou
au moins une des substances: acide citrique, acide succinique et acide pyruvique, et/ou
de la vitamine K$_1$.

13. Milieu suivant l'une quelconque des revendications 7 à 12, caractérisé en ce qu'il contient les constituants en un rapport quantitatif ajusté à la composition de plasma sanguin naturel.

14. Milieu suivant l'une quelconque des revendications 7 à 13, caractérisé en ce qu'il est exempt d'agents tensio-actifs, de sels de métaux lourds et de colorants.

15. Utilisation du milieu de culture cellulaire suivant l'une quelconque des revendications 7 à 14, pour la culture de cellules eucaryotiques, de préférence de leucocytes de mammifères.